# EUROPEAN PATENT SPECIFICATION

(11) **EP 1 616 008 B1**
(45) Date of publication and mention of the grant of the patent: **21.03.2007**
(21) Application number: 04727252.1
(22) Date of filing: 14.04.2004
(51) Int. Cl.: C12N 15/10, C12N 15/66, C12P 19/34, C12Q 1/68

(54) **LIGATION-BASED SYNTHESIS OF OLIGONUCLEOTIDES WITH BLOCK STRUCTURE**
LIGATIONS-BASIERENDE SYNTHESE VON OLIGONUCLEOTIDEN MIT BLOCKSTRUKTUREN
SYNTHESE PAR LIGATURE D'OLIGONUCLEOTIDES AVEC STRUCTURE EN BLOC

(30) Priority: 15.04.2003 EP 03008653
(43) Date of publication of application: 18.01.2006
(73) Proprietor: MAX-PLANCK-GESELLSCHAFT ZUR FÖRDERUNG DER WISSENSCHAFTEN E.V., 14195 Berlin (DE)
(72) Inventor: SOLDATOV, Aleksey, 12169 Berlin (DE); BORODINA, Tatiana, 12169 Berlin (DE); LEHRACH, Hans, 14129 Berlin (DE)
(74) Representative: Vossius & Partner
(86) International application number: PCT/EP2004/003921
(87) International publication number: WO 2004/092375

(56) References cited:
- EP-A- 1 327 682
- WO-A-00/29616
- WO-A-89/11211
- WO-A-97/42330
- WO-A-03/106637
- HAI-BAO CHEN ET AL: "A NEW METHOD FOR THE SYNTHESIS OF A STRUCTURAL GENE" NUCLEIC ACIDS RESEARCH, OXFORD UNIVERSITY PRESS, SURREY, GB, vol. 18, no. 4, 25 February 1990 (1990-02-25), pages 871-878, XP000099685 ISSN: 0305-1048
- "ENZYMATICALLY PRODUCED COMPOSITE PRIMERS: AN APPLICATION OF T4 RNA LIGASE-COUPLED PRIMERS TO PCR" BIOTECHNIQUES, EATON PUBLISHING, NATICK, US, vol. 19, no. 2, August 1995 (1995-08), page 182,184,186, XP001160946 ISSN: 0736-6205
- ANTSON D O ET AL: "PCR-generated padlock probes detect single nucleotide variation in genomic DNA." NUCLEIC ACIDS RESEARCH. 15 JUN 2000, vol. 28, no. 12, 15 June 2000 (2000-06-15), page E58, XP002302312 ISSN: 1362-4962 cited in the application
- MYER S E ET AL: "SYNTHESIS AND APPLICATION OF CIRCULARIZABLE LIGATION PROBES" BIOTECHNIQUES, EATON PUBLISHING, NATICK, US, vol. 30, no. 3, March 2001 (2001-03), pages 584-586,589, XP001149146 ISSN: 0736-6205 cited in the application
- BORODINA TATIANA A ET AL: "Ligation-based synthesis of oligonucleotides with block structure." ANALYTICAL BIOCHEMISTRY, vol. 318, no. 2, 15 July 2003 (2003-07-15), pages 309-313, XP002302313 ISSN: 0003-2697

### SEQUENCE LISTING

<110> Max-Planck-Gesellschaft zur Förderung der Wissenschaften e.V.
<120> Ligation-based synthesis of oligonucleotides with block structure
<130> G 2593 PCT
<140> PCT/EP2004/003921
   <141> 2004-04-14
<150> EP 03 00 8653.2
   <151> 2003-04-15
<160> 13
<170> PatentIn version 3.1
<210> 1
   <211> 44
   <212> DNA
   <213> artificial sequence
<220>
   <221> source
   <223> /note="Description of artificial sequence: central part of the padlock"
<400> 1
   ggaggttgcg aggcgtattc attgctcaga attcacgact cacg 44
<210> 2
   <211> 18
   <212> DNA
   <213> artificial sequence
<220>
   <221> source
   <223> /note="Description of artificial sequence: adapter for padlock preparation"
<400> 2
   cctcgcaacc tccggctc 18
<210> 3
   <211> 20
   <212> DNA
   <213> artificial sequence
<220>
   <221> source
   <223> /note="Description of artificial sequence: adapter for padlock preparation"
<400> 3
   cccgtcgtga gtcgtgaatt 20
<210> 4
   <211> 29
   <212> DNA
   <213> artificial sequence
<220>
   <221> source
   <223> /note="Description of artificial sequence: right variable part of the padlock'
<400> 4
   ttgtaaaacg tcgggagaaa cagagagcc 29
<210> 5
   <211> 23
   <212> DNA
   <213> artificial sequence
<220>
   <221> source
   <223> /note="Description of artificial sequence: left variable part of the padlock"
<400> 5
   acgggacatt taagaccaaa ctg 23
<210> 6
   <211> 52
   <212> DNA
   <213> artificial sequence
<220>
   <221> source
   <223> /note="Description of artificial sequence: region of genomic DNA polymorphic for accessions Columbia and Landsberg (variant for Columbia)"
<400> 6
   ctctctgttt ctcccgacgt tttacaacag tttggtctta aatgttcgcc gc 52
<210> 7
   <211> 50
   <212> DNA
   <213> artificial sequence
<220>
   <221> source
   <223> /note="Description of artificial sequence: region of genomic DNA polymorphic for accessions Columbia and Landsberg (variant for Landsberg)"
<400> 7
   tctctgtttc tcccgacgtt ttacaatagt ttggtcttaa atgttcgccg 50
<210> 8
   <211> 25
   <212> DNA
   <213> artificial sequence
<220>
   <221> source
   <223> /note="Description of artificial sequence: PCR primer with 4nt overhang"
<400> 8
   tatgaagcat atgccgagaa agatg 25
<210> 9
   <211> 28
   <212> DNA
   <213> artificial sequence
<220>
   <221> source.
   <223> /note="Description of artificial sequence: PCR primer with 4nt overhang"
<400> 9
   tatgtttgat tgtgaatgtg tcatcaac 28
<210> 10
   <211> 30
   <212> DNA
   <213> artificial sequence
<220>
   <221> source
   <223> /note="Description of artificial sequence: 5' part of composite PCR primer"
<400> 10
   ttttgtttaa ctttaagaag gagatataca 30
<210> 11
   <211> 28
   <212> DNA
   <213> artificial sequence
<220>
   <221> source
   <223> /note="Description of artificial sequence: 5' part of composite PCR primer"
<400> 11
   gatcctcagt ggtggtggtg gtggtgca 28
<210> 12
   <211> 21
   <212> DNA
   <213> artificial sequence
<220>
   <221> source
   <223> /note="Description of artificial sequence: adapter for preparation of composite PCR primers"
<400> 12
   catatgtata tctccttctt a 21
<210> 13
   <211> 14
   <212> DNA
   <213> artificial sequence
<220>
<221> source
   <223> /note=''Description of artificial sequence: adapter for preparation of composite PCR primers"
<400> 13
   catatgcacc acca 14

## Description

The present invention relates to a method of producing single-stranded nucleic acid molecules from oligo- or polynucleotides wherein each of said oligo- or polynucleotides has a predefined 5' or 3' terminus, comprising the steps of (a) annealing an adaptor oligonucleotide simultaneously or step by step to (aa) a first oligo- or polynucleotide; and (ab) a second oligo- or polynucleotide wherein the 5'-terminus of said adaptor oligonucleotide is complementary in sequence to the 5' terminus of said first oligo- or polynucleotide and the 3'-terminus of said adaptor molecule is complementary in sequence to the 3' terminus of said second oligo- or polynucleotide; and optionally (a') simultaneously with or subsequently to step (a) annealing at least one further adaptor oligonucleotide to free termini of said first or second oligonucleotides and to free termini of further oligo- or polynucleotides; (b) optionally filling in gaps between the neighbouring ends of said oligo- or polynucleotides; (c) ligating said oligo- or polynucleotides; and (d) removing said at least one adaptor oligonucleotide, wherein said single-stranded nucleic acid molecules represent a collection of nucleic acid molecules wherein either said first or said second oligo- or polynucleotide is invariable in sequence between all members of said collection of nucleic acid;molecules.

The invention is particularly efficient for the synthesis of long polynucleotides or sets of oligonucleotides with block structure.

As is known in the art, oligonucieotide-producing companies cannot guarantee a quantitative yield for oligos longer than 100 nucleotides (nt). Moreover, the yield and quality of the synthesis decrease dramatically when the length of oligonucleotide is more than 60 nt. The smallest possible scale of synthesis for 80-100mers is more then 200 nmol. Two-step purification (HPLC and PAGE) is required to obtain single-band oligonucleotides. The guaranteed output of purified 80-100mers is less than 1 nmol and the price is about 200-300 Euro.

Hai Bao Chen et al., NAR 18 (1990), 871-878 discloses a method of synthesizing a structural gene or gene fragment, consisting of the first synthesis of a single-stranded DNA (ssDNA). A plus-stranded DNA of the target gene was generally assembled by a stepwise or one step T4 DNA ligase reaction of six oligonucleotides (A, *pB, *pC, *pD, *pE and *pF) of 30 - 71 nucleotides long in the presence of two terminal complementary oligonucleotides (Ab' and eF') and three short interfragment complementary oligonucleotides (bc, cd and de). After purification, the synthetic ssDNA was inserted into a cloning vector, pWR13.

WO 97/42330 discloses the synthesis of composite nucleic acid molecules - by chain reaction cloning using nucleic acid molecules, a bridging oligo-nucleotide and a thermostable ligase. The composite nucleic acid molecule (NAM) was synthesized by ligating a first NAM to a second NAM, comprising: (a) maintaining a first NAM and a second NAM at a denaturing temperature; (b) forming a mixture comprising the first NAM and the second NAM with a bridging oligonucleotide (ON) and a thermostable ligase (TL), where the bridging ON comprises: (I) a 10-40 nucleotide sequence at its 5' end which is complementary to a nucleotide sequence on the 3' end of a strand of the first NAM; (II) a 10-40 nucleotide sequence at its 3' end which is complementary to a nucleotide sequence on the 5' end of a strand of a second NAM; where at annealing temperature, the bridging ON hybridises to sequences on the sense strand of both the first and second NAM or on the antisense strand of both the first and second NAM; (c) maintaining the mixture at an annealing temperature, whereby the bridging ON hybridises to sequences of both the first and second NAM and the TL ligates either the 3' end of the first NAM to the 5' end of the second NAM, or the 5' end of the first NAM to the 3' end of the second NAM to form a first strand of the composite NAM hybridised to a bridging ON; (d) maintaining the mixture at a denaturing temperature, whereby the bridging ON dissociates from the first strand of the composite NAM; and (e) maintaining the mixture at an annealing temperature, whereby the first strand of the composite NAM hybridises to complementary strands of the first NAM and the second NAM, where the TL ligates the end of the complementary strand of the first NAM to the end of the complementary strand of second NAM to form a second strand of the composite NAM.

WO 00/29616 discloses ligation assembly and detection of polynucleotides on solid supports allowing the creation of a single stranded polynucleotide of 50 to 500 nucleotides. Said method comprises: (a) annealing at least one bridging oligonucleotide and two or more assembly oligonucleotides such that a ligatable nick site is formed between adjacent assembly oligonucleotides, where one of the assembly oligonucleotides is immobilised to a solid support; (b) ligating the nick sites to form an immobilised single-stranded ligation product.

Biotechniques 19 (1995), 182, discloses enzymatically produced composite primers consisting of two parts: a gene-specific segment, which is unique for each primer, and an auxiliary segment (adaptor), which is usually the same for any particular application. The gene-specific and adaptor of a composite primer have distinct functions, and the problem of repeated incorporation of the same adaptor to different primers could be alleviated by synthesizing the adaptor segment separately and coupling it enzymatically to the gene-specific primer before e.g. PCR amplification. This document further describes the ligation of an 3' end of an adaptor to the 5' end of a phosphorylated 5' end of a second gene specific oligonucleotide and its use in PCR.

WO 89/11211 describes an oligonucleotide bank contains all conceivable (i.e. 4 to the power 6, 7, 8 or 9) different hexameric, heptameric, octameric or nonameric oligonucleotides. To sequence DNA, a single-stranded primer attachment site of known sequence is selected and to this site 2 immediately adjacent, hexa-, hepta- or octameric oligonucleotides are hybridised. Optional a third similar oligonucleotide is simultaneously, or subsequently, hybridised immediately adjacent to 1 of the other 2, and the oligomers ligated to form a primer. Particularly, the 2 oligomers are ligated to form a pre-primer, then the third hybridised and ligated with the pre-primer. The oligonucleotides are phosphorylated at the 5' end where this end is to be ligated to a neighbouring oligonucleotide using T4 ligase. After ligation and before sequencing, any non-ligated oligonucleotide is removed by heat-treatment.

None of the above documents anticipates the claimed invention or renders the claimed invention obvious.

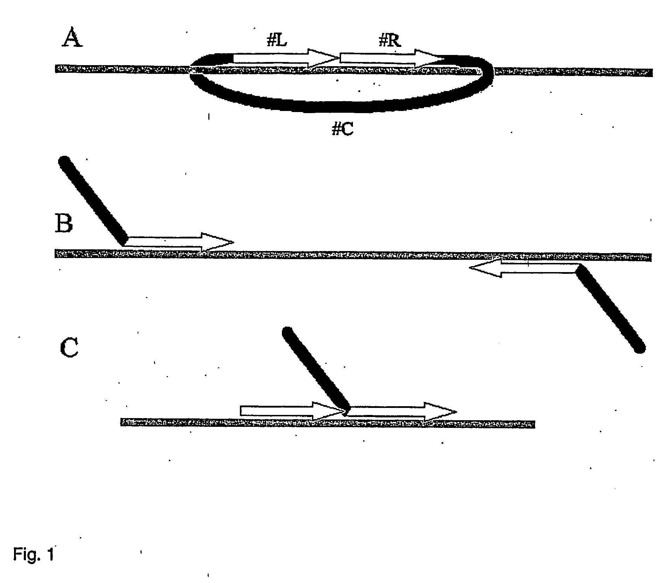

Oligonucleotides with block structure are widely used in molecular biology (Figure 1). Examples are: padlock probes (Lizardi et al. 1998; Pickering et al. 2002); primers with constant 5' regions, used for multiplex PCR amplification (Favis et al. 2000; Lindblad-Toh et al. 2000), ligase-independent cloning (de Costa and Tanuri 1998; Rashtchian et al. 1992; Zhou and Hatahet 1995; and commercial kits from Novagen, Invitrogen, BD Biosciences) and Invader assay (Mein et al. 2000).

Normally, these primers are synthesized by phosphoramidite technology and common regions have to be synthesized again and again in different oligonucleotides. It is expensive, especially, when the common part contains a hapten or fluorophore.

This problem becomes evident, for example, in the preparation of sets of padlock probes for SNP-detection projects. Padlock probes are typically 90-120nt long oligonucleotides, which consist of two locus-specific regions on both 3' and 5' ends connected by universal linker part (Figure 1A). The high price and the low yield of synthesis are the main obstacles for routine usage of padlock probes. Though they were shown to be an excellent tool for SNP detection and in situ localization, only few laboratories work with padlocks until now. Accordingly, the technical problem underlying the present invention was to provide methods for the quantitative and cost-sensitive production of single-stranded nucleic acid molecules that can in particular be employed as padlock probes.

The solution to said technical problem is achieved by providing the embodiments characterized in the claims. Thus, the present invention relates to a method of producing single-stranded nucleic acid molecules from oligo- or polynucleotides wherein each of said oligo- or polynucleotides has a predefined 5' or 3' terminus, comprising the steps of (a) annealing an adaptor oligonucleotide simultaneously or step by step to (aa) a first oligo- or polynucleotide; and (ab) a second oligo- or polynucleotide wherein the 5'-terminus of said adaptor oligonucleotide is complementary in sequence to the 5' terminus of said first oligo- or polynucleotide and the 3'-terminus of said adaptor molecule is complementary in sequence to the 3' terminus of said second oligo- or polynucleotide; and optionally (a') simultaneously with or subsequently to step (a) annealing at least one further adaptor oligonucleotide to free termini of said first or second oligonucleotides and to free termini of further oligo- or polynucleotides; (b) optionally filling in gaps between the neighboring ends of said oligo- or polynucleotides; (c) ligating said oligo- or polynucleotides; and (d) removing said at least one adaptor oligonucleotide, wherein said single-stranded nucleic acid molecules represent a collection of nucleic acid molecules and wherein either said first or said second oligo- or polynucleotide is invariable in sequence between all or essentially all members of said collection of nucleic acid molecules.

In accordance with the present invention, the term "oligonucleotide" refers to a uni-dimensional (i.e. not branched) stretch of nucleotides, preferably deoxyribonucleotides up to 30 nucleotides. The term also comprises oligonucleotides comprising or consisting totally of ribonucleotides. Also envisaged is that the oligonucleotides comprise unusual nucleotides such as unusual nucleotides as, for example, deoxyuridine, biotinylated or fluorescently labeled nucleotides, spacers or abasic residues. It is preferred that the oligonucleotide employed in the method of the invention consists of the four naturally occurring deoxyribonucleotides, i.e. adenine, cytosine, guanine and tymidine.

The term "polynucleotide" in accordance with the invention may consist of the same types of nucleotides that are described above for oligonucleotides.

However, a polynucleotide in accordance with the invention comprises a unidimensional stretch of at least 31 nucleotides.

The term "5'-terminus" refers to the 5'-terminal part of an oligo- or polynucleotide, preferably the terminal 5 or 4 nucleotides.

The term "complementary in sequence" refers to complementarity in sequence of at least 75% of the respective nucleotides, preferably at least 90% of the respective nucleotides and most preferred 100% of the respective nucleotides.

The term "essentially all members" refers to at least 90%, preferably at least 95%, more preferred at least 98% and most preferred to at least 99% such as 99.5% or 99.8% of all members.

In accordance with the present invention, a novel method of producing oligonucleotides by ligation of individual fragments by a ligase such as T4 DNA ligase is described. It is simple and allows the simultaneous processing of several reactions. The method is quantitative, cheap and does not require individual optimization. The possibility to purify products by HPLC makes the technology suitable for large-scale genomic projects. On the other hand, the same approach may be used for small-scale synthesis of composite primers for two-step PCR amplification and ligation-independent cloning. Small-scale reaction does not require any purification.

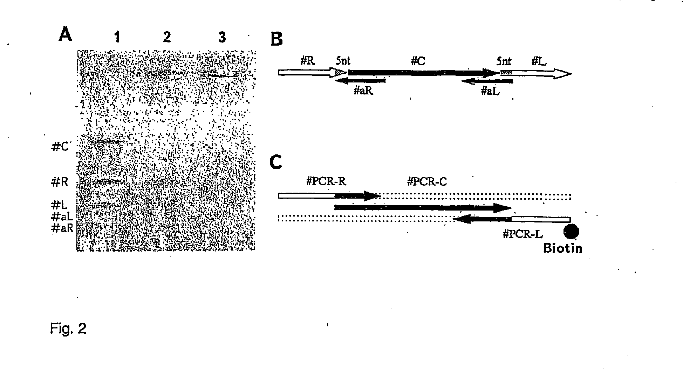

The method of the invention requires oligo- or polynucleotides having a predefined 5' or 3' terminus to which an adaptor polynucleotide which is complementary in sequence to, said predefined 5' or 3' terminus is annealed. Simultaneously or subsequently, the second oligo- or polynucleotide is annealed to said adapter oligonucleotide by way of complementarity of its 5' or 3' terminus. A schematic overview over the annealing process as provided in Fig. 2B wherein the first oligo- or polynucleotide may be represented by #R, the second oligo- or polynucleotide may be represented by #C and the adaptor oligonucleotide may be represented by #aR. Alternatively, the first oligonucleotide or polynucleotide may be represented by #C, the second oligonucleotide or polynucleotide may be represented by #L and the adaptor oligonucleotide may be represented by #aL.

The situation including optional step (a') is represented by the complete arrangement of oligonucleotides depicted in Fig. 2B. For example, if #R represents the first oligo- or polynucleotide and #C represents the second oligo- or polynucleotide and #aR represents the adaptor oligonucleotide, then #aL represents the further adaptor oligonucleotide and #L represents the further oligo- or polynucleotide.

If gaps are obtained after annealing of the adaptor oligonucleotide(s) to said first, second and optionally further oligo- or polynucleotide(s) then the gaps are filled in, for example, by polymerase activity such as T4 DNA polymerase activity. Subsequently, the at least two oligo- or polynucleotides are ligated using an appropriate ligase. Appropriate ligases depend, inter alia, on the-nature of the oligo- or polynucleotides used for preparation of the single-stranded nucleic acid molecules. For example, if the oligo- or polynucleotides are DNA, than it is preferred to use the T4 DNA ligase. Other ligases may also be used, for example thermostable commercially available Tth, Taq or Pfu ligases. Another possibility to perform ligation is a chemical template-dependent reaction (Xu and Kool 1999), which uses chemically activated oligonucleotides instead of enzyme..

Finally, the at least one adaptor oligonucleotide is removed. Removal can be effected by denaturated PAGE or chromatography, such as FPLC or HPLC. Other methods are known in the prior art comprising capture of biotine labeled adaptors or destruction of ribonucleotide adaptors by RNase (Nilsson et al. 2000).

The invention relates to, in other terms, a method of producing a collection of single-stranded nucleic acid molecules wherein each member of said collection of nucleic acid molecules comprises a portion that is invariable between all or essentially all members of said collection and at least one portion that is variable between different members of said collection and that is located 5' or 3' of said invariable portion, comprising the steps of (a) annealing at least one adaptor oligonucleotide, simultaneously or step by step to (aa) an oligo- or polynucleotide representing said invariable portion; and (ab) oligo- or polynucleotides representing said variable portions, wherein (i) a first part of said at least one adaptor oligonucleotide is complementary in sequence to the 5' terminus of said nucleic acid molecule representing said invariable portion and a second part of the at least one adaptor molecule is complementary in sequence to the 3' terminus of a nucleic acid molecule representing said variable portion; or (ii) a first part of said at least one adaptor oligonucleotide is complementary in sequence to the 3' terminus of said nucleic acid molecule representing said invariable portion and a second part of the at least one adaptor molecule is complementary in sequence to the 5' terminus of a nucleic acid molecule representing said variable portion; (b) optionally filling in gaps between the neighbouring ends of said invariable and said variable portions; (c) ligating the invariable and variable portions; and (d) removing said at least one adaptor oligonucleotide.

The above steps performed in accordance with the present invention per se can be effected by the person skilled in the art according to conventional protocols such as are provided in the appended examples. Temperature ranges include 4 to 42°C for annealing, fill-in reactions and ligation.

Reaction buffers include conventional reactions buffers such as disclosed, for example, in (Sambrook and Russell 2001).

Preferred is a method of the invention wherein the complementarity in sequence is at least four nucleotides such as 5, 6, 7, 8, 9 or 10 nucleotides. It is particularly preferred that the number of nucleotides which are complementary in sequence is five nucleotides. Also particularly preferred is that there is no mismatch within the stretch of complementarity.

In another preferred embodiment the invention relates to a method wherein annealing and ligation are simultaneously performed. Buffers can easily be adjusted to have annealing and ligation performed simultaneously. If these steps are performed simultaneously, then it is preferred that optional step (b) is omitted. The method of the invention can in this way be accelerated.

It is also preferred in accordance with the method of the present invention that the most valuable oligo- or polynucleotide in step (a) and/or (a') is provided in molar deficit relative of other oligo- and polynucleotides. The molar deficiency will guarantee that said oligo- or polynucleotide is consumed in the ligation reaction. The term "most valuable oligo- or polynucleotide" with respect to the present invention refers to invention refers to either (i) the most expensive oligonucleotide (labeled by hapten or fluorophore or the longest oligonucleotide) or (ii) oligonucleotide available in less quantity if compared with others.

In accordance with the invention it is further particularly preferred that said nucleic acid molecule representing said invariable portion is annealed with two adapter oligonucleotides, wherein further one of said adapter oligonucleotides is in a first part complementary in sequence with the 5' end of said nucleic acid molecule representing said invariable portion and the second adapter oligonucleotide is in a first part complementary to the 3' end of said nucleic acid molecule representing said invariable portion. In this embodiment, the respective termini of the adaptor polynucleotides not annealed to said invariable portion are annealed to termini of oligo- or polynucleotides representing variable portions of the single-stranded nucleic acid molecule. A schematic overview of such an arrangement is provided in Fig. 2B.

This embodiment of the method of the invention is particularly advantageous in the cost-sensitive and easy production, for example, padlock probes. It is also advantageous to use resulting single-stranded nucleic acid molecules in two-step PCR or ligase-independent cloning as will be discussed further below.

In principle, the nucleic acid molecules representing the variable portions may have at least one conserved terminus, namely the terminus that anneals to the adaptor oligonucleotide. In this case adaptor oligonucleotides may be essentially the same for the whole collection. Alternatively, the oligo- or polynucleotides representing variable portions may be without any conservative parts. Then the special adaptor oligonucleotide should be used for annealing of each particular nucleic acid molecule representing the variable portion. The terminus of said special adaptor oligonucleotide not annealed to the nucleic acid molecules representing the invariable portion must be predefined in order to allow a successful annealing reaction.

Most preferred is a method of the invention wherein the further oligo- or polynucleotides are variable in sequence between different members of said collection of nucleic acid molecules.

In accordance with the embodiments pertaining to the production of a collection of single-stranded nucleic acid molecules, it is finally preferred that the 5' or 3' termini of said oligo- or polynucleotides representing said variable sequences which anneal to said 5' or 3' termini of said adaptor oligonucleotide are invariable between different members of said oligo- or polynucleotides representing said variable sequences.
In another preferred embodiment of the method of the invention, ligation is effected with T4 DNA ligase. Most preferred is that about 1 unit of T4 DNA ligase is reacted in step (c) with about 4pmol of termini of the oligo- or polynucleotides annealed to said adaptor molecule(s). It is also preferred in this embodiment that the ligation reaction is carried out at a temperature of about 20°C. Ligation efficiency may significantly be increased if the reaction is carried out at, for example, 37°C-the temperature optimum for T4 DNA ligase. At this temperature, it is required that the complementary sequences comprise 5 or more nucleotides.

Further preferred is a method wherein the ligation reaction is carried out in the presence of some molecular crowding agent, for example, with at least 5% polyethylene glycol. The inclusion of polyethylene glycol above the indicated range is advantageous because it increases the ligation efficiency.

In accordance with this preferred embodiment, it is more preferred that the ligation reaction is carried out in the presence of 12 to 18% polyethylene glycol. It is particularly preferred that the ligation reaction is carried out in the presence of about 15% polyethylene glycol.

Preferred in accordance with the method of the invention is further that said polyethylene glycol is polyethylene glycol 6000.

In an additional preferred embodiment of the present invention, the method further comprises the step of purifying said single-stranded nucleic acid molecules. Purification can be performed according to standard protocols, see for example Sambrook, J., D. Russell. 2001. Molecular cloning: A laboratory manual. Cold Spring Harbor Laboratory, Cold Spring Harbor, NY.

Purification advantageously includes PAGE (Polyacrylamide Gel Electrophoresis) preferably under denaturing conditions, FPLC or HPLC or chromatography. Also preferred is an embodiment of the method of the invention further comprising modifying at least one of said oligo- or polynucleotides. In the alternative of the aforementioned embodiment, at least one of said oligo- or polynucleotides is modified when added to the reaction, i.e. the first step of the method of the invention.

In accordance with this preferred embodiment of the invention, the modification of the at least one oligo- or polynucleotide may be effected during one step of the method of the invention, for example when performing the fill-in reaction. Alternatively, a pre-modified oligonucleotide or polynucleotide may be included in the steps of the method of the invention. Modifications may be manifold and include the modifications recited herein below as being preferred.

Advantageously, the modification is a ribonucleotide, a spacer or a nucleotide comprising a detectable label.

Detectable labels include bioluminescent, phosphorescent, biotinilated, fluorescent and radioactive labels such as labels with ³²P or ³H.

In a particularly advantageous embodiment of the method, said oligo- or polynucleotides representing the invariable sequence are modified.

It is also preferred to automate the method of the invention. The ligation reaction may be assembled by liquid handling automated system. It is additionally preferred that the final product is purified by HPLC.

In an additional preferred embodiment of the method of the present invention, said method further comprises employing members of said collection of nucleic acid molecules in ligase-independent cloning (LIC). Composite primers for LIC have gene-specific 3'-parts and special 5'-parts (LIC system, Novagen; In-Fusion PCR cloning, BD Biosciences Clontech; Gateway PCR cloning system, Invitrogen).

**The figures show:**
**Figure 1:** Applications of oligonucleotides with block structure. Gene-specific parts are white, common parts are black. **A.** Template-dependent ligation of padlock probe. **B.** Primers for multiplex PCR amplification and ligation-independent cloning. **C.** Invader assay.
**Figure 2:** Padlock synthesis. **A.** Step by step PAGE analysis of padlock synthesis. Bands were visualized by UV shadowing as described in Methods. Lane 1 - unligated primers; lane 2 - result of ligation; lane 3 - PAGE-purifed padlock probe. Aliquots of the same reactions were taken for this gel. **B.** Scheme of ligation. **C.** PCR-based approach for padlock synthesis (Antson et al. 2000; Myer and Day 2001). Amplification is performed with two gene-specific primers having long 3' overhands (#PCR_ L and #PCR_R) on template #PCR_C. Single stranded padlock is purified after annealing to Streptavidine paramagnetic particles.

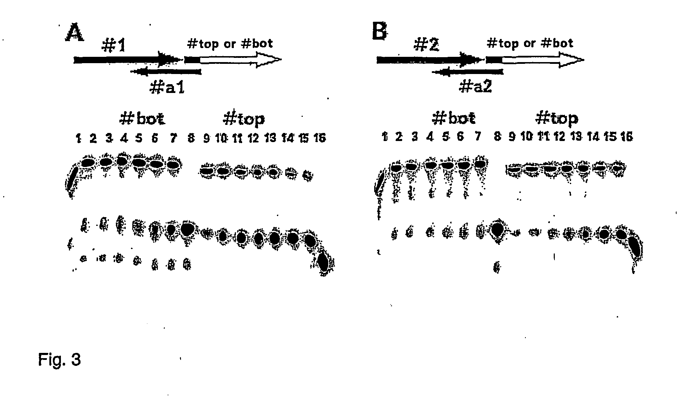

**Figure 3:** Ligation of different primers with the same 4nt overhangs. Ligation of [γ32P]ATP labeled #top and #bot primers with (#1; #a1) and (#2; #a2); see scheme under Table 1. Ligation was performed as described in Methods. Lanes 1-8: #bot primer; lanes 9-16: #top primer. Lanes (1-7) and (9-15) correspond to the sequential two times dilutions of T4 DNA ligase (400u for lanes 1 and 9). Lanes 8 and 16 - control without ligase. **A.** Ligation with #1 and #a1. **B.** Ligation with #2 and #a2.

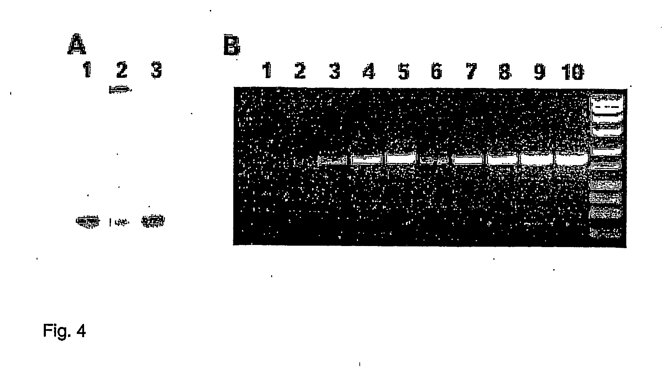

**Figure 4:** Application of ligated primers. **A.** Padlock probe circularizes only in the presence of perfectly matched template. Circular products have decreased mobility in PAGE comparing with linear ones. Lane 1-control without ligase; 2 - ligation on #T1 (perfectly matched) template; 3 - ligation on #T2 (mismatched) template. **B.** PCR amplification of ORF of phi29 polymerase (1,7kb) with composite and gene-specific primers. Lines 1-5 - composite primers (#1&#top and #2&#bot); lines 6-10 - gene-specific primers (#top and #bot). Lanes 1 and 6 - after 12 cycles; lanes 2 and 7 - after 16 cycles; lanes 3 and 8 - after 20 cycles; lanes 4 and 9 - after 24 cycles; lanes 5 and 10 - after 28 cycles. M - marker.

### MATERIALS AND METHODS

Oligonucleotides were synthesized by TIB Molbiol (Berlin, Germany). Primer sequences are given in Table 1. T4 DNA ligase and T4 PNK were from New England BioLabs (Beverly, USA). Tfh ligase was from ABgene (UK).

Polyacrylamide gels with radiolabeled' oligonucleotides were exposed on a Fuji imaging Plate without any fixation. For long exposition (more than couple of hours) cassette with gel was freezed at -20°C. Freezing prevents diffusion of even 4nt long oligonucleotides.

### Padlock probes.

Phosphorylation of #C and #L oligonucleotides was performed at 37°C for 1 hour. 1nmol of primer was incubated in 10µl of T4 PNK buffer (TrisHCl, pH 7.6 70mM; MgCl₂ 1.0mM; DTT 5mM) with 1 mM ATP and 2.5u of T4 PNK followed by enzyme heat inactivation at 65°C for 20 minutes. Phosphorylated primers were used in ligation reactions without purification.

The scheme of the ligation based synthesis of padlock probe is shown on Figure 2B. 200pmol-scale ligation reaction was performed for 1 hour at 20°C in 20µl of mixture: 1xT4 ligase buffer (TrisHCl, pH 7.5 50mM; MgCl₂ 10mM; DTT 10mM; ATP 1mM; BSA 25µg/ml); PEG 6000 15%; T4 DNA ligase 100u. To ensure, that all #C oligonucleotides are consumed in annealing and ligation reactions, adaptors and locus-specific primers were taken in a slight excess relative to the common primer: #C - 200pmol; ##aR=#aL - 220pmol; #R=#L - 240pmol (1:1,1:1,2). In some experiments adaptors were annealed to the common primer before addition of enzyme and locus-specific primers (heating the mixture to 90°C and cooling gradually to normal temperature), but this measure is not essential for the method of the invention.

Padlock probes may be phosphorylated directly in the ligation mixture after heat inactivation of T4 DNA ligase (e. g. 65°C for 15 minutes).

Padlocks were purified through denaturing PAGE electrophoresis. The corresponding band was visualized by UV shadowing on the DC Alufolien Kiselgel 60F254 (Merck, Germany) chromatographic plate (or on printer paper with a somewhat lower sensitivity) and was cut out. DNA was ethanol precipitated after elution from the gel in 150µl of (TrisHCl, pH 7.5 10mM; EDTA 1 mM; NaCl 200mM) for 1 hour at 60°C.

Circularization of 40fmol of a [γ-32P]ATP labeled padlock probe on 2fmol of matched or mismatched synthetic template was performed in 10µl of 1xTth ligation buffer (TrisHCl, pH 8.3 20mM; MgCl₂ 10mM; KCI 50mM; EDTA 1mM; NAD⁺ 1 mM; DTT 10mM; Triton X-100 0,1%) by 1u of Tth ligase for 20 cycles of (94°C for 20 sec and 60°C for 3 min).

### Composite primers for PCR.

Ligation was performed separately for (#top, #1, #a1) and (#bot, #2, #a2) primer sets: 1 hour at 20°G and 20 min at 70°C in 10µl of mixture: 1xT4 ligase buffer; PEG 6000 15%; T4 DNA ligase 400u; phosphorylated #top or #bot primers - 20pmol; #a1 or #a2-25pmol; #1 or #2 - 30pmol. Composite primers were used in PCR amplification without any purification.

Amplification was performed in 50µl with Advantage cDNA polymerase Mix (Clontech, USA). 1µl of phage phi29 suspension (5x10¹⁰ 1/ml; DSMZ, Germany) was used as a template. Parameters of PCR with #top and #bot primers were: 10pmol of both primers; 96°C 2min (95°C 20sec, 62°C 20sec, 68°C 1min20sec)x28 cycles. PCR with composite primers: 1pmol of both composite primers, 10pmol of #1 and #2 primers; 96°C 2min, (95°C 20sec, 62°C 20sec, 68°C 1min20sec)x5 cycles, (95°C 20sec, 58°C 20sec, 68°C 1min20sec)x23 cycles. Two different annealing temperatures were used for amplification with composite primers because melting temperature of external primer #1 (59.6°C) was less, than that of internal primers #top and #bot (65.°C).

The examples illustrate the invention.

### Example 1: Padlock synthesis.

Padlock probes (padlocks) are typically 90-120nt oligonucleotides, which consist of two locus-specific regions on both 3' and 5' ends connected by universal linker part (Figure 1A). The ability of padlocks for template-dependent circularization is used for in situ localization and SNP detection (Antson et al. 2000; Lizardi et al. 1998; Myer and Day 2001; Pickering et al. 2002). High quality of locus-specific ends is important in ligation reaction, because they should create a nick with perfect base pairing.

The scheme of the ligation-based synthesis of padlocks is shown on Figure 2B. Adaptor primers #aL and #aR and central part primer #C (all shown black) are common for the whole set of padlocks. Primers #R and #L (shown white) are locus-specific. 5nt 3' and 5' overhangs of adaptor primers serve for ligation of locus-specific primers. Small excess of adapters (1,1x) and locus-specific primers (1,2x) guarantee that all #C oligonucleotides will be consumed in ligation. The scheme is practically the same for synthesis of oligos with common 3' or 5' regions (just one adaptor and one locus-specific primer instead of two).

Melting temperatures of overlaps of adaptor primers with common primer are about 50 °C in the ligation mixture. In principle, it is possible to use shorter #C-complementary segments of adaptor primers thus decreasing the price of the procedure. Overhangs for locus-specific primers were selected to be 5nt long (see scheme below Table 1). Such a length was satisfactory for the purpose of the present invention. However, longer overhangs showed better ligation efficiency (data not presented). Moreover, for longer overhangs it is possible to increase the ligation efficiency to about twice by carrying out the reaction at 37°C - i.e. the optimal temperature for T4 DNA ligase.

The method of the invention requires attention to the selection of primer ends (termini) to avoid misligation. In accordance with the present invention it was found that due to the high fidelity of T4 DNA ligase this restriction is not stringent. To estimate the efficiency of mismatch ligation, we have compared ligation of 3' overhangs (3'-CTCGG...5') with perfectly matched primers/oligonucleotides (5'-...GAGCC-3') and with two mismatched primers/oligonucleotides: (5'-...GAGga-3') and (5'-...GAGCg-3'). In experiments with ten times excess of ligase we have not detected any ligation products for overhangs containing one and two mismatched bases. No problems with misligation were observed for overhangs shown in Table 1. It is possible to improve the ligation accuracy by increasing the concentration of monovalent ions in reaction buffer (Wu and Wallace 1989), and to exclude any participation of adaptor oligos in ligation by blocking their 3' ends.

According to the present New England BioLabs Catalogue, 1 unit of T4 DNA ligase is capable to join about 1,2 pmol of nicks of λ/Hind III digested DNA in 1 hour. In a test ligation of #C with #R and #aR we have obtained a similar result: 1u - 0,4pmol. Because PEG 6000 increases the ligation efficiency (Pheiffer and Zimmerman 1983), we have checked the influence of 5% - 20% PEG 6000 on the reaction. 15% PEG turned out to be the most effective, increasing the ligation efficiency about 15 times: 1 u - 6pmol. For padlock synthesis, 0.5 unit of T4 DNA ligase should be added per 1 pmol of the #C primer. This ratio was determined in a series of small-scale ligations with decreasing amounts of the enzyme (data not shown) and agrees well with titration on individual nicks.

Padlock probes were purified in denaturing PAGE (Figure 2A). The yield is quantitative: 70% according to spectrophotometer measurements and close to 100% according to PAGE (Figure 2A). It seems that some UV adsorbing impurities are removed on this step. Purified padlock probes run as single bands in denaturing PAGE (Figure 2A) and are suitable for SNP-discriminating ligation (Figure. 4A). PAGE purification is the most time-consuming and laborious step of the procedure. However, in conventional phosphoramidite synthesis this step also cannot be avoided (see below). Besides, in ligation-based procedures distinct differences between the length of padlock and initial primers (Figure 2A) - allows to use HPLC purification instead of PAGE. Adaptor primers do not participate in ligation and may be repurified together with padlocks in large-scale projects.

Ligation-based procedure is superior if compared with the conventional phosphoramidite synthesis.

**Length restriction.** Oligonucleotide-producing companies cannot guarantee a quantitative yield for oligos longer than 100nt and do not take orders for primers longer than 130nt. In contrast, the ligation-based method of the present invention is only restricted by the synthesis of individual components: for 3-component padlock probe the procedure is practical up to 150-180nt.

**Price and quality**. According to information from all three companies listed in Table 2, a two-step purification (HPLC and PAGE) is required for 80-100nt long oligonucleotides. HPLC alone cannot separate full-length oligonucleotides from nearby contaminants. Smallest-scale synthesis of one 100nt oligonucleotide costs about 170 Euro and gives about 3 nmol of product after HPLC purification (Table 2A). Judging from Operon the yield after PAGE purification will be less than 1 nmol.

5 nmol - scale synthesis of single padlock oligonucleotide by ligation method costs 120 Euro (Table 2B). In our hands the yield after PAGE purification is more then 3 nmol. Comparing "170 Euro per 1 nmol" with "120 Euro per 3 nmol", the ligation-based synthesis of the present invention is four times cheaper. What is more important, the quality of ligase-based synthesis is higher. It provides bands practically without "n-1" contaminants. In principle, HPLC purification may be used instead of PAGE.

**Synthesis of large sets**. For producing a set of oligonucleotides with block structure by the phoshoramidite technology, common regions have to be synthesized- again-and again. The- price per oligo does not change if compared with the single synthesis. For example, synthesis of 40 100nt padlocks for 20 SNP loci (two padlocks per loci) by the conventional procedure costs 6720 Euro (Table 2A). In the ligation-based method the price per oligonucleotide drops dramatically, because common parts need to be synthesized only once. The same set costs 1846 Euro. The price difference is 10 fold (.6720 Euro, 1 nmol of each padlock; 1846 Euro - 3 nmol).

If common parts contain biotin or fluorescein, conventional synthesis will be 2000 Euro more expensive (40 padlocks x 50 Euro) and the yield drops about two fold. Ligation-based synthesis will cost 100 Euro more (2 padlocks x 50 Euro) and the yield remains the same.

Two PCR based methods of padlock synthesis were suggested recently (Antson et al. 2000; Myer and Day 2001; and Figure 2C), but they have some disadvantages if compared with proposed ligation-based technology. Locus-specific primers have long overhangs for PCR initiation (-12nt (Antson et al. 2000) and -18nt (Myer and Day 2001)). It is impossible to insert modified bases in some definite position during PCR. Using of proofreading polymerase (Antson et al. 2000) results in heterogeneous 3' ends of padlocks. Finally, single-strand purification by streptavidine PMP's is expensive (about 1ml of Dynabeads is required for 500pmol of padlock).

To summarise, the ligation-based technology in accordance with the present invention permits to synthesize parts of composite oligonucleotides in separate reactions of the appropriate scale. Waste is minimal. Adaptor primers may be reused. Synthesis may be automated, because it is compatible with HPLC-based purification. For large sets of composite oligonucleotides the price per one primer tends almost equals the price of locus-specific parts and becomes comparable with that of 40-50mers, which are widely used now in molecular biology.

### Example 2: Composite primers for PCR amplification.

Composite primers with gene-specific 3' regions (Figure 1B) are used for multiplex PCR amplification (Favis et al. 2000; Lindblad-Toh et al. 2000; Eurogenetics - genome primer sets), ligase-independent cloning (LIC) and Invader assay (Mein et al. 2000). They have "block structure": 3' parts (17-25nt) identify gene target and 5' regions define some particular application: second-stage PCR (16-20nt), concrete LIC scheme (LIC system, Novagen - 15nt; In-Fusion PCR cloning, BD Biosciences Clontech - 16nt; Gateway PCR cloning system, Invitrogen - 29nt) and so on.

Some applications require combination of blocks. For example, the same gene-specific part should be attached to different vector-specific parts for optimization of the protein expression (Dieckman et al. 2002). Frequently, the required amount of primers is very small. Only one successful PCR reaction is necessary for LIC or for preparation of genome-sequencing tags (Eurogenetics: http://www.eurogentec.com/code/en/geno_dnaa_prod.htm).

Each composite primer should be synthesized individually by conventional phosphoroamidite technology. Moreover, decreasing of the synthesis scale does not decrease the price of the primers proportionally. Some LIC methods require insertion of modified bases in composite oligonucleotides, for example 4-5 dUracils (Rashtchian et al. 1992) or 2-3 phosphorothioate bonds (de Costa and Tanuri 1998; Zhou and Hatahet 1995). In this case the price of composite oligos increases in 2-3 times.

PCR amplification is more difficult with long composite primers. Sometimes, the only way to obtain single-band product is to use cloned or preliminarily amplified fragments as a template. This means that two sets of primers should be used in this case: gene-specific pair and composite pair.

It is very convenient to prepare small amounts of composite primers by attaching presynthesized blocks to each other. In this case it is possible to combine gene-specific parts with different 5' parts.

Small-scale procedure for preparation of composite primers from individual blocks with the help of T4 RNA ligase was described previously (Kaluz et al. 1995). This enzyme needs no overhangs for joining of two oligos. The only disadvantage of the method is that some part of the reaction products (depends on excess of external primer) have duplication of gene-specific 3' part. It is not critical for some applications, but can provide problems for cloning. Another disadvantage is that phosphorylation and ligation should be performed separately.

In contrast to the above recited prior art approaches, the (T4 DNA) ligase based method of the present invention requires overhangs for ligation, but guarantees the homogeneity of the product. Ligated primers may be used in PCR without any purification because PEG and other components of ligation mixture do not inhibit PCR. Ligation and phosphorilation may be performed simultaneously in a ligation buffer (result not shown).

Adaptor primers do not interfere with PCR. To decrease the risk of false priming by adaptor primers and mis-annealing of long composite primers it is most appropriate to use a mixture of external and composite primers (in molar ratio 10:1) instead of composite primers alone in an amplification reaction.

An example of amplification with composite primers produced by ligation is shown in Figure 4B. The ORF of phi 29 polymerase (Blanco and Salas 1996) was amplified from 1µl of phage culture. In both cases the required products were obtained. PCR was slower with composite primers if compare with gene specific ones. Worse kinetics at least partly depends on external primers, because the amplification is more effective with-(#top and #bot) pair than with (#1 and #2) pair (results not shown).

There are two possible schemes of design of adaptor primers for ligation. One approach is to order individual adaptor for each particular combination of 5' and 3' parts. Adaptors should be 10-14nt long (two 5-7nt overlaps). They are cheaper than long composite primers. The price of external primers should be left out of account, because they may be used with a number of gene-specifc primers. A 20pmol aliquot of HPLC purified external primer costs less then 10 Euro-cent

Another approach is to use the standard adaptors and to prepare gene-specific primers with predefined overhangs (as in the method for padlock preparation, see above). Long overhangs may conflict with some cloning schemes, but too short overhangs may be a bar for the effective ligation. G/C-reach 5nt overhangs (5'-...GAGCC-3') and (5'-ACGGG...-3') are sufficient for effective ligation (see padlock preparation above). To obtain an idea about the suitability of shorter sequences we have tried to use the (5'-TATG...-3') sequence for the preparation of composite primers. This sequence was selected because ATG may be combined with the first Met codon of the open reading frame. It turns out, that 4nt AIT-rich overhang requires much more ligase. Moreover, different amounts of enzyme were necessary for different combinations of primers (Figure 3). Nevertheless, 1µl (400u) of T4 ligase was enough to prepare 20pmol of composite primers. It costs about 1 Euro, much less, if compared with the price of long composite primers.

### Conclusion.

Ligation-based technology is based on construction of composite oligonucleotides from individual blocks. Here we have demonstrated, how this method may be applied for two different tasks:
(i) preparative-scale production of padlock probes;
(ii) -small-scale synthesis of PCR primers.

Preparative-scale procedure gives products of high quality. In contrast to the conventional phosphoroamidite synthesis, HPLC may be used instead of PAGE purification.

Small-scale procedure is fast one-tube reaction. It does not require any purification and may be automated.

In both cases the suggested method is considerably cheaper if compared with the conventional phosphoramidite synthesis. The pride difference increases at least two fold, when oligonucleotides contain modified bases.

### REFERENCES

**Antson, D.O., A. Isaksson, U. Landegren, M. Nilsson.** 2000. PCR-generated padlock probes detect single nucleotide variation in genomic DNA. Nucleic Acids Res. *28(12)*: e58.
**Blanco, L., M. Salas.** 1996. Relating structure to function in phi29 DNA polymerase. J Biol Chem. *271(15)*:8509-8512.
**da Costa, L.J., A. Tanuri**. 1998. Use of T7 gene 6 exonuclease and phosphorothioated primers for the manipulation of HIV-1 infectious clones. J Virol Methods. *72(1)*:117-21.
**Dieckman, L., M. Gu, L. Stols, M.I. Donnelly, F.R. Collart.** 2002. High throughput methods for gene cloning and expression. Protein Expr Purif. *25(1)*:1-7.
**Favis, R., J.P. Day, N.P. Gerry; C. Phelan, S. Narod, F. Barany. 2000.** Universal DNA array detection of small insertions and deletions in BRCA1 and BRCA2. Nat Biotechnol. *18*:561-564.
**Kaluz, S., M. Kaluzova, A.P. Flint.** 1995. Enzymatically produced composite primers: an application of T4 RNA ligase- coupled primers to PCR. Biotechniques. *19(2)*:182-4, 186.
**Lindblad-Toh, K., E. Winchester, M.J. Daly, D.G. Wang, J.N. Hirschhorn, J.P. Laviolette, K. Ardlie, D.E. Reich, E. Robinson, P. Sklar, N. Shah, D. Thomas, J.B. Fan, T. Gingeras, J. Warrington, N. Patil, T.J. Hudson, E.S. Lander**. 2000. Large-scale discovery and genotyping of single-nucleotide polymorphisms in the mouse. Nat Genet. *24*:381-386.
**Lizard!, P.M., X. Huang, Z. Zhu, P. Bray-Ward, D.C. Thomas, D.C. Ward.** 1998. Mutation detection and single-molecule counting using isotherma) rolling-circle amplification. Nat Genet. *19*:225-232.
**Mein, C.A., B.J. Barratt, M.G. Dunn, T. Siegmund, A.N. Smith, L. Esposito, S. Nutland, H.E. Stevens, A.J. Wilson, M.S. Phillips, N. Jarvis, S. Law, M. de Arruda, J.A. Todd. 2000**. Evaluation of single nucleotide polymorphism typing with invader on PCR amplicons and its automation. Genome Res. *10*:330-343.
**Myer, S.E., D.J. Day.** 2001. Synthesis and application of circularizable ligation probes. Biotechniques. *30*:584-593.
**Nilsson, M., G. Barbany, D-O. Antson, K. Gertow, U.Landegren.** 2000. Enhanced detection and distinction of RNA by enzymatic probe ligation. Nature biotechnology. 18:791-793
**Pheiffer, B.H., S.B. Zimmerman.** 1983. Polymer-stimulated ligation: enhanced blunt- or cohesive-end ligation of DNA or deoxyribooligonucleotides by T4 DNA ligase in polymer solutions. Nucleic Acids Res. *11*:7853-7871.
**Pickering, J., A. Bamford, V. Godbole, J. Briggs, G. Scozzafava, P. Roe, C. Wheeler, F. Ghouze, S. Cuss**. 2002. Integration of DNA ligation and rolling circle amplification for the homogeneous, end-point detection of single nucleotide polymorphisms. Nucleic Acids Res. *30*:e60.
**Rashtchian, A., G.W. Buchman, D.M. Schuster, M.S. Berninger**. 1992. Uracil DNA glycosylase-mediated cloning of polymerase chain reaction-amplified DNA: application to genomic and cDNA cloning. Anal Biochem. *206(1)*:91-7.
**Sambrook, J., D. Russell.** 2001. Molecular cloning: a laboratory manual. Cold Spring Harbor Laboratory. Cold Spring harbor, NY.
**Wu, . D.Y., R.B. Wallace.** 1989. Specificity of the nick-closing activity of bacteriophage T4 DNA ligase. Gene. *76*:245-254.
**Xu, Y., E.T. Kool.** 1999. High sequence fidelity in a non-enzymatic DNA autoligation reaction. Nucleic Acid Res. 27(3):875-81.
**Zhou, M., Z. Hatahet.** 1995. An improved ligase-free method for directional subcloning of PCR amplified DNA. Nucleic Acids Res. *23(6)*:1089-90.

### TABLES

**Table 1. Oligonucleotide sequences.**

| SNP positions in template primers (T1 and T2) are in bold. Alignments for ligation-based synthesis of padlock and PCR primers are shown under the table. Overhangs for ligation are in bold. | |
|---|---|
| #C | GGAGGTTGCGAGGCGTATTCATTGCTCAGAATTCACGACTCACG |
| #aR | CCTCGCAACCTCCGGCTC |
| #aL | CCCGTCGTGAGTCGTGAATT |
| #R | TTGTAAAACGTCGGGAGAAACAGAGAGCC |
| #L | ACGGGACATTTAAGACCAAACTG |
| #T1 | CTCTCTGTTTCTCCCGACGTTTTACAACAGTTTGGTCTTAAATGTTCGCCGC |
| #T2 | TCTCTGTTTCTCCCGACGTTTTACAATAGTTTGGTCTTAAATGTTCGCCG |
| | |
| #top | TATGAAGCATATGCCGAGAAAGATG |
| #bot | TATGTTTGATTGTGAATGTGTCATCAAC |
| #1 | TTTTGTTTAACTTTAAGAAGGAGATATACA |
| #2 | GATCCTCAGTGGTGGTGGTGGTGGTGCA |
| #a1 | CATATGTATATCTCCTTCTTA |
| #a2 | CATATGCACCACCA |

**Allignment of oligonucleotides for preparation of padlock:**
#R 5'-TTGTA&AACGTC.GGGASA&ACAGAGAGCC-3' 5' -ACGGGACATTTAAGACCAAACTG-3' #L
5'-GGAGGTT-GCGAGGCGTATTCATTGCTCAGAATTCACGACTCACG-3' #C
#aR 3'-CTCGGCCTCCAACGCTCC-5' 3'- TTAAGTGCTGAGTGCTGCCC-5' #aL

**Table 2. Synthesis of 100nt primer.**

| **(A) Conventional method.** | | | | |
|---|---|---|---|---|
| **Company** | **Synthesis scale** | **Price per base** | **Purification** | **Guaranteed yield and price** |
| **Operon** | 11µmol | 2.05 Euro | HPLC&PAGE (118 Euro) | 0.5nmol 323 Euro |
| **MWG** | >0.2 µmol | 1.78 Euro | HPSF included | 3 nmol HPSF* 178 Euro |
| **TIB** | >0.2 µmol | 1.68 Euro | HPLC included | 3 nmol HPLC* 1.68 Euro |

| | | | | |
|---|---|---|---|---|
| *- both companies recommend to perform additional PAGE purification in laboratory. **Operon:** QIAGEN Operon GmbH, Cologne, Germany; **MWG:** MWG Biotech, Ebersberg, Germany **TIB:** TIB MOLBIOL, Berlin, Germany | | | | |

| **(B) Ligation-based method** | | |
|---|---|---|
| (prices are given according to TIB). | | |
| **Component** | **Scale and purification** | **Guaranteed yield and price** |
| #L, #R | up to 30 bases long, HPLC purified | 5 nmol; 39.08 Euro |
| #aL, #aR | up to 20 bases long, standard purification | 5 nmol; 19.98 Euro |
| #C | up to 60 bases long, standard purification | 5 nmol; 55.00 Euro |
| T4 DNA ligase | 1250u | 3.25 Euro |
| T4 PNK | 25u | 2.50 Euro |
| | | |
| Total: | | 5nmol 120 Euro |

**Table 3. Ligation-based synthesis of 40 100nt padlocks for 20 SNP loci (two padlocks per one locus).**

| **Component** | **Scale and purification** | **Price per 20 loci** (Euro) |
|---|---|---|
| #L1, #L2, #R | 5 nmol of each, HPLC purified | 1172 |
| #aL and #aR | 500 nmol of each*, standard purification | 114 |
| #C1, #C2 | 250 nmol of each *, standard purification | 330 |
| T4 DNA ligase | 50000u | 130 |
| T4 PNK | 1000u | 100 |
| **Total:** | | 1846 Euro |
| **Per one padlock:** | 5nmol | 46.2 Euro |

| | | |
|---|---|---|
| *Two times more, than is required for 40 padlocks. | | |

## Claims

1. A method of producing single-stranded nucleic acid molecules from oligo- or polynucleotides wherein each of said oligo- or polynucleotides has a predefined 5' or 3' terminus, comprising the steps of
(a) annealing an adaptor oligonucleotide simultaneously or step by step to
(aa) a first oligo- or polynucleotide; and
(ab) a second oligo- or polynucleotide
wherein the 5'-terminus of said adaptor oligonucleotide is complementary in sequence to the 5' terminus of said first oligo- or polynucleotide and the 3'-terminus of said adaptor molecule is complementary in sequence to the 3' terminus of said second oligo- or polynucleotide;
(b) ligating said oligo- or polynucleotides; and
(c) removing said at least one adaptor oligonucleotide,
wherein said single-stranded nucleic acid molecules represent a collection of nucleic acid molecules and wherein either said first or said second oligo- or polynucleotide is invariable in sequence between all members of said collection of nucleic acid molecules.

2. The method of claim 1 additionally comprising:
(a') simultaneously with or subsequently to step (a) annealing at least one further adaptor oligonucleotide to free termini of said first or second oligonucleotides and to free termini of further oligo- or polynucleotides.

3. The method of claim 1 or 2 additionally comprising prior to step (b)
(b') filling in gaps between the neighbouring ends of said oligo- or polynucleotides.

4. The method of any one of claims 1 to 3 wherein the complementarity in sequence is at least four nucleotides.

5. The method of any one of claims 1 to 4 wherein annealing and ligation are simultaneously performed.

6. The method of any one of claims 1 to 5 wherein the adaptor oligonucleotide(s) in step (a) and/or (a') is/are provided in molar excess over the first or second or further oligo- or polynucleotides.

7. The method of any one of claims 1 to 6 wherein said first or said second oligo- or polynucleotide which is not invariable is variable in sequence between different members of said collection of nucleic acid molecules.

8. The method of any one of claims 2 to 7 wherein the further oligo- or polynucleotides are variable in sequence between different members of said collection of nucleic acid molecules.

9. The method of any one of claims 1 to 8 wherein the oligo- or polynucleotides representing said variable sequences are provided in molar excess over the nucleic acid molecule representing said invariable sequences.

10. The method of any one of claims 1 to 9 wherein the 5' or 3' termini of said oligo- or polynucleotides representing said variable sequences which anneal to said 5' or 3' termini of said adaptor oligonucleotide are invariable between different members of said oligo- or polynucleotides representing said variable sequences.

11. The method of any one of claims 1 to 10 where ligation is effected with T4/DNA ligase.

12. The method of any one of claims 1 to 11 wherein the ligation reaction is carried out in the presence of at least 5% polyethylene glycol.

13. The method of claim 12 wherein the ligation reaction is carried out in the presence of about 15% polyethylene glycol.

14. The method of claim 12 or 13 wherein said polyethylene glycol is polyethylene glycol 6000.

15. The method of any one of claims 1 to 14 wherein about 1 unit of T4 DNA ligase is reacted in step (b) with about 4 pmol of termini of the oligo- or polynucleotides annealed to said adaptor molecule(s).

16. The method of any one of claims 1 to 13 further comprising the step of purifying said single-stranded nucleic acid molecules.

17. The method of claim 16 wherein purification includes PAGE electrophoresis, HPLC or chromatography.

18. The method of any one of claims 1 to 17 further comprising modifying at least one of said oligo- or polynucleotides.

19. The method of any one of claims 1 to 18 wherein at least one of said oligo- or polynucleotides is modified.

20. The method of claim 18 or 19 wherein the modification is a ribonucleotide, a spacer or a nucleotide comprising a detectable label.

21. The method of any one of claims 18 to 20 wherein said oligo- or polynucleotides representing the invariable sequence are modified.

22. The method of any one of claims 1 to 21 further comprising employing members of said collection of nucleic acid molecules in the determination of SNPs in vitro.

23. The method of any one of claims 1 to 21 further comprising employing members of said collection of nucleic acid molecules in ligase-independent cloning or two-step PCR.

## Patentansprüche

1. Verfahren zur Herstellung einzelsträngiger Nukleinsäuremoleküle aus Oligo- oder Polynukleotiden, wobei jedes der Oligo- oder Polynukleotide ein vorbestimmtes 5'- oder 3'-Ende aufweist, umfassend die Schritte
(a) gleichzeitige oder schrittweise Aneinanderlagerung eines Adaptor-Oligonukleotids an
(aa) ein erstes Oligo- oder Polynukleotid; und
(ab) ein zweites Oligo- oder Polynukleotid,
wobei das 5'-Ende des Adaptor-Oligonukleotids in seiner Sequenz komplementär zu dem 5'-Ende des erstes Oligo- oder Polynukleotids und das 3'-Ende des Adaptormoleküls in seiner Sequenz komplementär zum 3'-Ende des zweiten Oligo- oder Polynukleotids ist;
(b) Ligation der Oligo- oder Polynukleotide; und
(c) Entfernen des mindestens einen Adaptor-Oligonukleotids, wobei die einzelsträngigen Nukleinsäuremoleküle eine Sammlung von Nukleinsäuremolekülen darstellen und wobei entweder das erste oder das zweite Oligo- oder Polynukleotid in seiner Sequenz unter allen Mitgliedern des Sammlung an Nukleinsäuremolekülen nicht variabel ist.

2. Verfahren nach Anspruch 1, zusätzlich umfassend:
(a') Aneinanderlagerung von mindestens einem weiteren Adaptor-Oligonukleotid an freie Enden des ersten oder zweiten Oligonukleotids und an freie Enden weiterer Oligo- oder Polynukleotide, gleichzeitig oder nach Schritt (a).

3. Verfahren nach Anspruch 1 oder 2, zusätzlich umfassend vor Schritt (b)
(b') Auffüllen von Lücken zwischen den benachbarten Enden der Oligo- oder Polynukleotide.

4. Verfahren nach einem der Ansprüche 1 bis 3, wobei die Komplementarität in der Sequenz mindestens vier Nukleotide beträgt.

5. Verfahren nach einem der Ansprüche 1 bis 4, wobei die Aneinanderlagerung und Ligation gleichzeitig durchgeführt werden.

6. Verfahren nach einem der Ansprüche 1 bis 5, wobei das Adaptor-Oligonukleotid/die Adaptor-Oligonukleotide in Schritt (a) und/oder (a') in einem molaren Überschuss gegenüber den ersten oder zweiten oder weiteren Oligo- oder Polynukleotiden bereitgestellt wird/werden.

7. Verfahren nach einem der Ansprüche 1 bis 6, wobei das erste oder zweite Oligo- oder Polynukleotid, welches nicht nicht variabel ist, in der Sequenz zwischen verschiedenen Mitgliedern der Sammlung an Nukleinsäuremolekülen variabel ist.

8. Verfahren nach einem der Ansprüche 2 bis 7, wobei die weiteren Oligo- oder Polynukleotide in der Sequenz zwischen verschiedenen Mitgliedern der Sammlung von Nukleinsäuremolekülen variabel sind.

9. Verfahren nach einem der Ansprüche 1 bis 8, wobei die Oligo- oder Polynukleotide, welche die variablen Sequenzen darstellen, in molarem Überschuss gegenüber den die nicht variablen Sequenzen darstellenden Nukleinsäuremolekülen bereitgestellt werden.

10. Verfahren nach einem der Ansprüche 1 bis 9, wobei die 5'- oder 3'-Enden der Oligo- oder Polynukleotide, welche die sich an die 5'- oder 3'-Enden der Adaptor-Oligonukleotide anlagernden variablen Sequenzen darstellen, unter den verschiedenen Mitgliedern der die variablen Sequenzen darstellenden Oligo- oder Polynukleotide nicht variabel sind.

11. Verfahren nach einem der Ansprüche 1 bis 10, wobei die Ligation durch die T4-DNA-Ligase bewirkt wird.

12. Verfahren nach einem der Ansprüche 1 bis 11, wobei die Ligationsreaktion in Gegenwart von mindestens 5% Polyethylenglycol durchgeführt wird.

13. Verfahren nach Anspruch 12, wobei die Ligationsreaktion in Gegenwart von etwa 15% Polyethylenglycol durchgeführt wird.

14. Verfahren nach Anspruch 12 oder 13, wobei das Polyethylenglycol Polyethylenglycol 6000 ist.

15. Verfahren nach einem der Ansprüche 1 bis 14, wobei etwa eine Einheit T4-DNA-Ligase in Schritt (b) mit etwa 4pMol Enden der an das Adaptormolekül/die Adaptormoleküle angelagerten Enden der Oligo- oder Polynukleotide umgesetzt wird.

16. Verfahren nach einem der Ansprüche 1 bis 13, weiter umfassend den Schritt der Reinigung der einzelsträngigen Nukleinsäuremoleküle.

17. Verfahren nach Anspruch 16, wobei die Aufreinigung PAGE-Elektrophorese, HPLC oder Chromatographie einschließt.

18. Verfahren nach einem der Ansprüche 1 bis 17, weiter umfassend die Modifizierung von mindestens einem der Oligo- oder Polynukleotide.

19. Verfahren nach einem der Ansprüche 1 bis 18, wobei mindestens eines der Oligo- oder Polynukleotide modifiziert ist.

20. Verfahren nach Anspruch 18 oder 19, wobei die Modifikation ein Ribonukleotid, ein Spacer oder ein eine nachweisbare Markierung umfassendes Nukleotid ist.

21. Verfahren nach einem der Ansprüche 18 bis 20, wobei die die nicht variablen Sequenzen darstellenden Oligo- oder Polynukleotide modifiziert sind.

22. Verfahren nach einem der Ansprüche 1 bis 21, weiter umfassend den Einsatz von Mitgliedern der Sammlung an Nukleinsäuremolekülen bei der Bestimmung von SNPs in vitro.

23. Verfahren nach einem der Ansprüche 1 bis 21, weiter umfassend den Einsatz von Mitgliedern der Sammlung an Nukleinsäuremolekülen in Ligaseunabhängiger Klonierung oder Zwei-Schritt-PCR.

## Revendications

1. Procédé de production de molécules d'acide nucléique simple brin provenant d'oligo- ou de polynucléotides, dans lequel chacun desdits oligo- ou polynucléotides a une extrémité 5' ou 3' prédéfinie, comprenant les étapes consistant à :
(a) hybrider un oligonucléotide adaptateur simultanément ou étape par étape à
(aa) un premier oligo- ou polynucléotide ; et
(ab) un second oligo- ou polynucléotide
l'extrémité 5' dudit oligonucléotide adaptateur étant complémentaire en termes de séquence à l'extrémité 5' dudit premier oligo- ou polynucléotide et l'extrémité 3' de ladite molécule adaptateur est complémentaire en termes de séquence à l'extrémité 3' dudit second oligo- ou polynucléotide ;
(b) lier lesdits oligo- ou polynucléotides; et
(c) retirer ledit au moins un oligonucléotide adaptateur,
dans lequel lesdites molécules d'acide nucléique simple brin représentent une collection de molécules d'acide nucléique et dans lequel soit ledit premier soit ledit second oligo- ou polynucléotide est invariable en termes de séquence entre tous les membres de ladite collection de molécules d'acide nucléique.

2. Procédé selon la revendication 1 comprenant de plus :
(a') de manière simultanée ou ultérieure à l'étape (a), l'hybridation d'au moins un autre oligonucléotide adaptateur aux extrémités libres desdits premier ou second oligonucléotides et aux extrémités libres d'autres oligo- ou polynucléotides.

3. Procédé selon la revendication 1 ou 2 comprenant de plus avant l'étape (b)
(b') le remplissage des brèches entre les extrémités avoisinantes desdits oligo- ou polynucléotides.

4. Procédé selon l'une quelconque des revendications 1 à 3, dans lequel la complémentarité en termes de séquence est d'au moins quatre nucléotides.

5. Procédé selon l'une quelconque des revendications 1 à 4, dans lequel l'hybridation et la liaison sont effectuées simultanément.

6. Procédé selon l'une quelconque des revendications 1 à 5, dans lequel le ou les oligonucléotides adaptateurs dans l'étape (a) et/ou (a') est/sont mis à disposition en excès molaire par rapport au premier ou au second ou à d'autres oligo- ou polynucléotides.

7. Procédé selon l'une quelconque des revendications 1 à 6, dans lequel ledit premier ou ledit second oligo- ou polynucléotide qui n'est pas invariable, est variable en termes de séquence entre différents membres de ladite collection de molécules d'acide nucléique.

8. Procédé selon l'une quelconque des revendications 2 à 7, dans lequel les autres oligo- ou polynucléotides sont variables en termes de séquence entre différents membres de ladite collection de molécules d'acide nucléique.

9. Procédé selon l'une quelconque des revendications 1 à 8, dans lequel les oligo- ou polynucléotides représentant lesdites séquences variables sont mis à disposition en excès molaire par rapport à la molécule d'acide nucléique représentant lesdites séquences invariables.

10. Procédé selon l'une quelconque des revendications 1 à 9, dans lequel les extrémités 5' ou 3' desdits oligo- ou polynucléotides représentant lesdites séquences variables qui effectuent une hybridation desdites extrémités 5' ou 3' dudit oligonucléotide adaptateur sont invariables entre différents membres desdits oligo- ou polynucléotides représentant lesdites séquences variables.

11. Procédé selon l'une quelconque des revendications 1 à 10, dans lequel la liaison est effectuée avec une T4/ADN ligase.

12. Procédé selon l'une quelconque des revendications 1 à 11, dans lequel la réaction de liaison est réalisée en présence d'au moins 5 % de polyéthylèneglycol.

13. Procédé selon la revendication 12, dans lequel la réaction de liaison est réalisée en présence d'environ 15 % de polyéthylèneglycol.

14. Procédé selon la revendication 12 ou 13, dans lequel ledit polyéthylèneglycol est du polyéthylèneglycol 6000.

15. Procédé selon l'une quelconque des revendications 1 à 14, dans lequel environ 1 unité d'ADN ligase T4 réagit dans l'étape (b) avec environ 4 pmol d'extrémités des oligo- ou polynucléotides hybridées à ladite/auxdites molécules adaptateur.

16. Procédé selon l'une quelconque des revendications 1 à 13, comprenant en outre l'étape consistant à purifier lesdites molécules d'acide nucléique simple brin.

17. Procédé selon la revendication 16, dans lequel la purification comporte une électrophorèse sur gel de polyacrylamide (PAGE), une chromatographie liquide à haute pression (HPLC) ou une chromatographie.

18. Procédé selon l'une quelconque des revendications 1 à 17, comprenant en outre la modification d'au moins un desdits oligo- ou polynucléotides.

19. Procédé selon l'une quelconque des revendications 1 à 18, dans lequel au moins un desdits oligo- ou polynucléotides est modifié.

20. Procédé selon la revendication 18 ou 19, dans lequel la modification est un ribonucléotide, un espaceur ou un nucléotide comprenant une étiquette détectable.

21. Procédé selon l'une quelconque des revendications 18 à 20, dans lequel lesdits oligo- ou polynucléotides représentant la séquence invariable sont modifiés.

22. Procédé selon l'une quelconque des revendications 1 à 21, comprenant en outre l'emploi des membres de ladite collection de molécules d'acide nucléique dans la détermination de SNPs in vitro.

23. Procédé selon l'une quelconque des revendications 1 à 21, comprenant en outre l'emploi de membres de ladite collection de molécules d_{'}acide nucléique dans un clonage indépendant d'une ligase ou une PCR à deux étapes.
